# EUROPEAN PATENT SPECIFICATION

(11) **EP 1 758 888 B1**
(45) Date of publication and mention of the grant of the patent: **17.02.2010**
(21) Application number: 05748329.9
(22) Date of filing: 27.05.2005
(51) Int. Cl.: C07D 401/12, A61K 31/454, A61P 25/28

(54) **BUTYRYLCHOLINESTERASE SELECTIVE INHIBITORS**
BUTYRYLCHOLINESTERASESELEKTIVE INHIBITOREN
INHIBITEURS DE BUTYRYLCHOLINESTÉRASE SELECTIVE

(30) Priority: 28.05.2004 EP 04076585
(43) Date of publication of application: 07.03.2007
(73) Proprietor: Neuropharma S.A.U., 28760 Tres Cantos (Madrid) (ES)
(72) Inventor: MARTÍNEZ GIL, Ana, E-28760 Tres Cantos - Madrid (ES); RUBIO ARRIETA, Laura, E-28760 Tres Cantos - Madrid (ES); MUÑOZ RUÍZ, Pilar, E-28760 Tres Cantos - Madrid (ES); DORRONSORO DÍAZ, Isabel, E-28760 Tres Cantos - Madrid (ES); GARCÍA PALOMERO, Esther, E-28760 Tres Cantos - Madrid (ES); DEL MONTE MILLÁN, María, E-28760 Tres Cantos - Madrid (ES); MEDINA PADILLA, Miguel, E-28760 Tres Cantos - Madrid (ES)
(74) Representative: ABG Patentes, S.L.
(86) International application number: PCT/EP2005/005816
(87) International publication number: WO 2005/118570

(56) References cited:
- EP-A- 0 229 391
- EP-A- 1 300 395
- WO-A-03/068220

## Description

### FIELD OF THE INVENTION

The present invention relates to indolylpiperidine compounds having selective pharmacological activity towards the butyrylcholinesterase (BuChE) enzyme, to processes of preparation of such compounds, to pharmaceutical compositions comprising them, and to their use in therapy, in particular for the treatment and or prophylaxis of a disease in which BuChE is involved, such as cognitive and neurodegenerative disorders.

### BACKGROUND OF THE INVENTION

Alzheimer's Disease (AD) is a common progressive dementia involving loss of memory and higher cognitive function. The disease is characterized by the presence of amyloid deposits in the brains of sufferers. These deposits are found both extracellularly (amyloid plaques) and intracellularly (neurofibrillary tangles). The principal constituent of amyloid plaques is the amyloid protein (Aβ) which is produced by proteolytic cleavage for the amyloid protein precursor (APP). The principal constituent of neurofibrillary tangles is the cytoskeletal protein tau.

Acetylcholinesterase (EC 3.1.1.7; AChE) and butyrylcholinesterase (EC 3.1.1.8; BuChE) are two closely homologous proteins. Both are present in all vertebrates, and both are capable of hydrolyzing the neurotransmitter, acetylcholine (ACh). In human, the two functionally distinct cholinesterases, AChE and BuChE, which share a high degree of amino acid sequence homology (>50%), are encoded by two separate genes, AChE and BChE, respectively. The two genes have similar exon-intron organization but radically different nucleotide composition, AChE being G,C-rich while BChE is A,T-rich. The presence of two distinct ChE genes in all vertebrates studied to date, indicates that both protein products are biologically required in these species, and presumably that they have distinct roles.

It has been suggested that anti-cholinergic drugs impair the memory of healthy individuals in a manner parallel to that observed early in the development of AD. Therefore, the principal current AD therapeutic approach, and the most promising one in the short term, is the stimulation of the cholinergic system. Since the "cholinergic hypothesis" of Alzheimer's Disease (AD) was established, the understanding of cholinergic mechanisms in this disorder has evolved. Cholinesterases (ChE) inhibitors are currently the main pharmacological approach to treatment, offering a rational evidence-based approach to symptom management (Giacobini E., Neurochem Res. 2003 Apr; 28(3-4):515-22. "Cholinesterases: new roles in brain function and in Alzheimer's Disease"). However, the anti-ChE agent, physostigmine, has been shown to have a small, short-term positive effect on cognitive functions. Precursor loading, with choline or phosphatidyl choline, is ineffective.

Originally, research also focused on selective acetylcholinesterase (AChE) inhibitors. Although overlooked for many years, butyrylcholinesterase (BuChE) is also capable of hydrolysing acetylcholine (ACh) and may play an important role in the pathophysiology and symptomatology of AD (Greig NH, Lahiri DK, Sambamurti K, Int. Psychogeriatr. 2002, 14:77-91 "Butyrylcholinesterase: an important new target in Alzheimer's Disease therapy").

The observation that BuChE becomes associated with plaques at the point when they mature from a diffuse, benign form to the compact neurotoxic form associated with pathological disease, has led to the suggestion that BuChE may play an active role in this process (Saez-Valero J, et al., J Neurosci Res. 2003 72(4):520-6 "Glycosylation of acetylcholinesterase and butyrylcholinesterase changes as a function of the duration of Alzheimer's Disease").

The content of BuChE in the brain increases with age, whereas that of AChE displays a reverse trend. The catalytic activity of BuChE may therefore play a more prominent role in ACh hydrolysis in the aging brain, suggesting that the inhibition of BuChE may have a greater impact on cholinergic neurotransmission in the elderly. The presence of BuChE in the amyloid plaques and neurofibrillary tangles of AD has been now confirmed by numerous investigators. It seems reasonable to assume that this enzyme is a glial product and that its location in the plaques and tangles may result from the overall inflammatory processes related to AD. Interfering with the activity of this enzyme (catalytic or non-catalytic) represents a promising therapeutic strategy to influence the course of the neuropathological processes in AD (Greig NH, Utsuki T, Yu Q, Zhu X, Holloway HW, Perry T, Lee B, Ingram DK, Lahiri DK. Curr Med Res Opin. 2001; 17(3):159-65, "A new therapeutic target in Alzheimer's Disease treatment: attention to butyrylcholinesterase"; Giacobini E. Drugs Aging., 2001, 18(12): 891-8, "Selective inhibitors of butyrylcholinesterase: a valid alternative for therapy of Alzheimer's Disease?").

Piperidine derivatives are compounds of interest in the pharmaceutical industry, many families within this class have given a variety of biological activities associated with the CNS, such as inhibition of AChE or hydroxytryptamine (HT) receptors.

EP 229 391 discloses piperidine derivatives having selective anti-AChE activity. They present an aromatic moiety, indol is disclosed among others. EP 1 300 395 describes 4-substituted piperidine compounds having AChE inhibitory action.

CN 1345724 discloses indolylpiperidines for the treatment of Alzheimer's Disease, the compounds disclosed have AChE inhibitor activity but they are not selective for BuChE (see CN 1345724, table 1).

WO03068220 discloses 1-arylsulfonyl-3-substituted indole or indoline derivatives displaying binding to the 5-HT6 receptor. The disclosed compounds are described to be useful for the treatment of central nervous disorders, specifically Alzheimer's and Parkinson's Disease, among others.

Despite the potential therapeutic applications of antagonists of BuChE, to date very few compounds with selective BuChE inhibition activity have been reported, such as for example ethopropazine (10-(2-diethylaminopropyl) phenothiazine hydrochloride), dansylarginine N-(3-ethyl-1,5-pentanediyl)amide (DAPA), phenethylnorcymserine and compounds disclosed in WO 9902154 or EP1251131. Clearly, there is a need to find compounds that have pharmacological activity towards BuChE, being both highly effective and selective, able to differentiate between BuChE and AChE.

### SUMMARY OF THE INVENTION

We have now found a family of structurally distinct class of compounds which are very selective inhibitors of BuChE, some of them showing activities even below the nanomolar range. Further, the compounds of the invention have low toxicity.

Structural important features of the compounds is the presence of an heterocyclic moiety, a piperidine moiety with an aralkyl substituent on the N, and a linker between these two moieties, the linker containing an amide-like functionality. We have found that selectivity and activity can be modulated with the nature and length of the linker, and the nature and substituents of the above mentioned moieties. It is important for the selectivity that the heterocycle is not linked to the linker via a carbonyl or heteroatom.

In one aspect the invention is directed to a compound of the formula I: 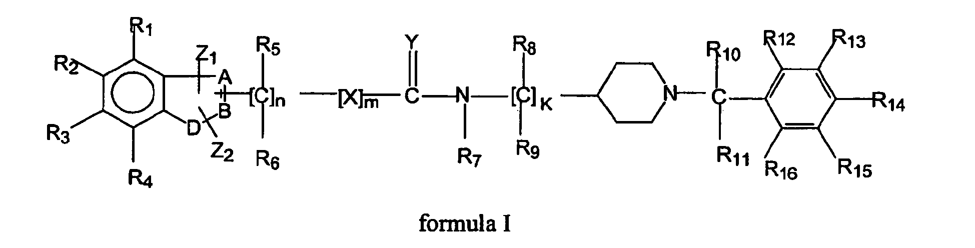 wherein
A,B are independently selected from C or N;
D is selected from C, O, S, N;
with the proviso that at least one of A, B and D is an heteroatom;
X is selected from -CRₐR_{b}-, -O-, -S-, -NRₐ-;
Y is selected from O, S, NRₐ;
Z₁ and Z₂ are independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, -CORₐ, -C(O)ORₐ,-C(O)NRₐR_{b}, -C=NRₐ, -CN, -ORₐ, -OC(O)Rₐ, -S(O)ₜ-Rₐ, -NRₐR_{b}, -NRₐC(O)R_{b}, -NO₂,-N=CRₐR_{b}, or halogen,
wherein Z₁ and Z₂ together with A and B or B and D can form a fused ring system, or together with R₁ or R₄ they can form a fused ring system;
R₁ to R₁₆ are independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, -CORₐ, -C(O)ORₐ,-C(O)NRₐR_{b}, -C=NRₐ, -CN, -ORₐ, -OC(O)Rₐ, -S(O)ₜ-Rₐ, -NRₐR_{b}, -NRₐC(O)R_{b}, -NO₂,-N=CRₐR_{b} or halogen;
wherein Rₐ and R_{b} are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy or halogen;
n is 1 to 6;
m is 0 or 1;
k is 1-8;
or a pharmaceutically acceptable salt or solvate thereof;
with the proviso that the compound is not (aR) 1H-Indole-3-propanamide, N-[[1-[(4chlorophenyl)methyl]-4piperidinyl]methyl]-a-[(3-ethoxybenzoyl)amino].

The compound (aR) 1H-Indole-3-propanamide, N-[[1-[(4chlorophenyl)methyl]-4piperidinyl]methyl]-a-[(3-ethoxybenzoyl)amino] is disclosed in WO 9925686.

In another aspect the invention is directed to pharmaceutical compositions which comprise a compound according to formula (I) or a pharmaceutically acceptable salt or solvate thereof, and a pharmaceutically acceptable carrier, adjuvant or vehicle. In a preferred embodiment the formulation is oral.

The present invention is also directed to the use of the above defined compounds in the manufacture of a medicament, preferably for the treatment of cognitive disorders as senile dementia, cerebrovascular dementia, mild recognition impairment, attention deficit disorder, and /or neurodegenerative dementing disease with aberrant protein aggregations as specially Alzheimer's Disease or condition, or prion disease as Creutzfeld-Jakob disease or Gerstmann-Straussler-Scheinker disease.

In another aspect, the invention is directed to the use of the above defined compounds as reactives for biological assays.

In another aspect the invention is directed to a process for preparing a compound of formula I above by amidation of a reagent containing the heterocycle moiety with an amine containing the 4 -substituted piperidine moiety.

### DETAILED DESCRIPTION OF THE INVENTION

The typical compounds of this invention selectively inhibit butyrylcholinesterase, with a clear difference in inhibition of AChE of some orders of magnitude as shown by the

### examples.

In the above definition of compounds of formula (I) the following terms have the meaning indicated:
"Alkyl" refers to a straight or branched hydrocarbon chain radical consisting of carbon and hydrogen atoms, containing no saturation, having one to eight carbon atoms, and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents such as a halo, hydroxy, alkoxy, carboxy, cyano, carbonyl, acyl, alkoxycarbonyl, amino, nitro, mercapto and alkylthio.
"Amino" refers to a radical of the formula-NH₂, -NHRₐ or -NRₐR_{b}, wherein Rₐ and R_{b} are as defined above.
"Aryl" refers to a phenyl, naphthyl, indenyl, fenanthryl or anthracyl radical, preferably phenyl or naphthyl radical. The aryl radical may be optionally substituted by one or more substituents such as hydroxy, mercapto, halo, alkyl, phenyl, alkoxy, haloalkyl, nitro, cyano, dialkylamino, aminoalkyl, acyl and alkoxycarbonyl, as defined herein.
"Aralkyl" refers to an aryl group linked to an alkyl group. Preferred examples include benzyl and phenethyl.
"Acyl" refers to a radical of the formula-C(O)-R_{c} and-C (O)-R_{d} where R_{c} is an alkyl radical as defined above and R_{d} is an aryl radical as defined above, e. g., acetyl, propionyl, benzoyl, and the like.
"Cycloalkyl" refers to a stable 3-to 10-membered monocyclic or bicyclic radical which is saturated or partially saturated, and which consist solely of carbon and hydrogen atoms. Unless otherwise stated specifically in the specification, the term "cycloalkyl" is meant to include cycloalkyl radicals which are optionally substituted by one or more substituents such as alkyl, halo, hydroxy, amino, cyano, nitro, alkoxy, carboxy and alkoxycarbonyl.
"Fused aryl" refers to an aryl group, especially a phenyl or heteroaryl group, fused to another ring.
"Alkoxy" refers to a radical of the formula-ORa where Ra is an alkyl radical as defined above, e. g., methoxy, ethoxy, propoxy, etc.
"Halo" refers to bromo, chloro, iodo or fluoro.
"Heterocyclyl" refers to a stable 3-to 15 membered ring radical which consists of carbon atoms and from one to five heteroatoms selected from the group consisting of nitrogen, oxygen, and sulfur, preferably a 4-to 8-membered ring with one or more heteroatoms, more preferably a 5-or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.

References herein to substituted groups in the compounds of the present invention refer to the specified moiety that may be substituted at one or more available positions by one or more suitable groups, e. g., halogen such as fluoro, chloro, bromo and iodo; cyano; hydroxyl; nitro; azido; alkanoyl such as a C1-6 alkanoyl group such as acyl and the like; carboxamido; alkyl groups including those groups having 1 to about 12 carbon atoms or from 1 to about 6 carbon atoms and more preferably 1-3 carbon atoms; alkenyl and alkynyl groups including groups having one or more unsaturated linkages and from 2 to about 12 carbon or from 2 to about 6 carbon atoms; alkoxy groups having one or more oxygen linkages and from 1 to about 12 carbon atoms or 1 to about 6 carbon atoms; aryloxy such as phenoxy; alkylthio groups including those moieties having one or more thioether linkages and from 1 to about 12 carbon atoms or from 1 to about 6 carbon atoms; alkylsulfinyl groups including those moieties having one or more sulfinyl linkages and from 1 to about 12 carbon atoms or from 1 to about 6 carbon atoms; alkylsulfonyl groups including those moieties having one or more sulfonyl linkages and from 1 to about 12 carbon atoms or from 1 to about 6 carbon atoms; aminoalkyl groups such as groups having one or more N atoms and from 1 to about 12 carbon atoms or from 1 to about 6 carbon atoms; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

We prefer that in the compounds of formula I the linker contains alkylene units between the moieties, such as in the compounds of following formula II: 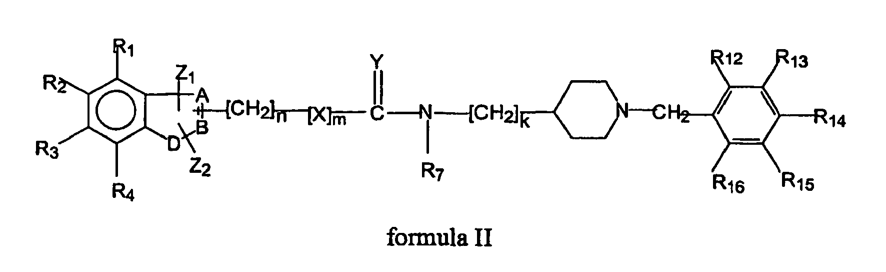 wherein A,B,D, Z₁, Z₂, X, Y, n, m, k, and R₁ to R₁₆ are as defined above.

In another embodiment we prefer that in the compounds of formula I the linker contains an amide functionality.

In a further embodiment the heterocyclic moiety is an indol-like heterocycle, such as in the compounds represented by formula III: 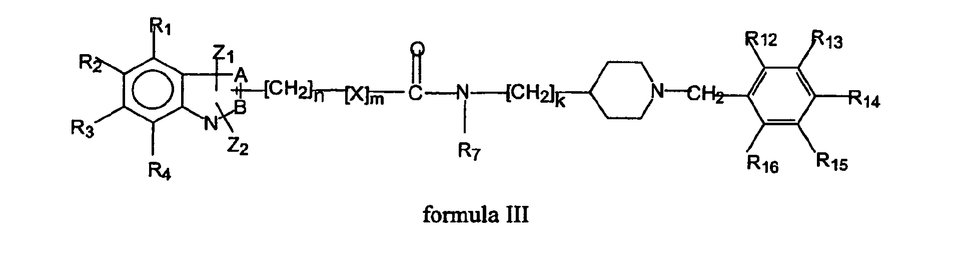 wherein A, B, Z₁, Z₂, X, n, m, k, and R₁ to R₁₆ are as defined. The N of the heterocycle can be further substituted, for example with alkyl, aralkyl, etc.

In another embodiment the piperidine is N substituted by a benzyl group. In this case the compounds are preferably represented by formula IV: 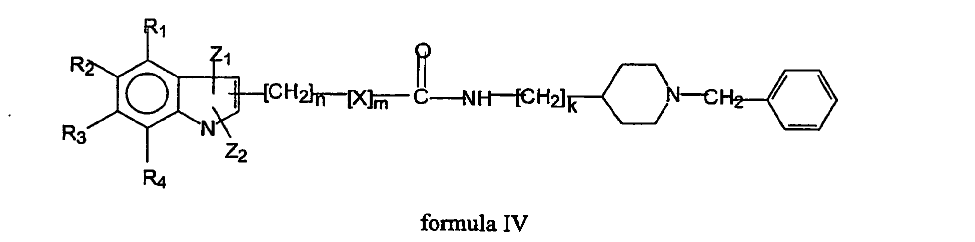 wherein Z₁, Z₂, X, n, m, k, and R₁ to R₄ are as defined.
In this case the indol heterocycle is preferably linked to the linker at position 3. In a preferred variant which shows a particularly good selectivity and activity, m is 0 and n is 2.

In a further embodiment X is preferably -CH₂- or -O-.

In another embodiment k is preferably 2.
Some compounds of the invention are given in the examples, among them compound 5 is preferred due to its good activity and selectivity.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The term "pharmaceutically acceptable salts, solvates" refers to any pharmaceutically acceptable salt, or solvate which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts can be carried out by methods known in the art.

For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

The compounds of the invention may be in crystalline form either as free compounds or as solvates and it is intended that both forms are within the scope of the present invention. Methods of solvation are generally known within the art. Suitable solvates are pharmaceutically acceptable solvates. In a particular embodiment the solvate is a hydrate.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts or solvates.

The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

The compounds of formula (I) defined above can be obtained by available synthetic procedures, from fragments containing the heterocycle and the piperidine moieties and the linking them to form the desired linker between them, such as : 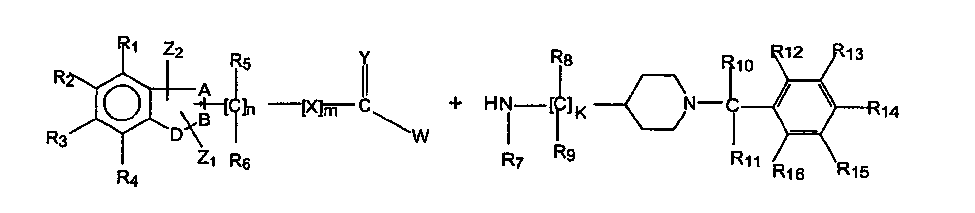 wherein the substituents are as defined above and W is a leaving group.

For example, they can be prepared by amidation of indole carboxylic acid derivatives with amino piperidine compounds, such as described in Padwa. A. et al, Synthesis, 9, 1994, 993-1004. General method for the synthesis of carbamates are described for example in Bruce, A.; Spangle, L. A.; Kaldor, S. W.; Tetrahedron Letters, 1996, 7, 937-940.

The reaction products may, if desired, be purified by conventional methods, such as crystallisation or chromatography. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

One preferred pharmaceutically acceptable form is the crystalline form, including such form in a pharmaceutical composition. In the case of salts and solvates the additional ionic and solvent moieties must also be non-toxic. The compounds of the invention may present different polymorphic forms, it is intended that the invention encompasses all such forms.

The compounds represented by the above-mentioned formula (I) of the present invention, a salt thereof or a solvate of them exhibit a superior butyrylcholinesterase inhibitory action. Therefore, another aspect of this invention relates to the use of said compounds in the manufacture of a medicament for treating, improving or preventing an BuChE related disease or condition which method comprises administering to a patient in need of such a treatment a therapeutically effective amount of a compound of formula (I) or a pharmaceutical composition thereof. Among the diseases that can be treated are cognitive disorders as senile dementia, cerebrovascular dementia, mild recognition impairment, attention deficit disorder, and /or neurodegenerative dementing disease with aberrant protein aggregations as specially Alzheimer's Disease or condition, or prion disease as Creutzfeld-Jakob disease or Gerstmann-Straussler-Scheinker disease.

The present invention further provides pharmaceutical compositions comprising a compound of this invention, or a pharmaceutically acceptable salt or stereoisomers thereof together with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.
The compounds and compositions of this invention may be used with other drugs to provide a combination therapy. The other drugs may form part of the same composition, or be provided as a separate composition for administration at the same time or at different time.

The following examples are given as further illustration of the invention, they should not be taken as a definition of the limits of the invention.

### EXAMPLES

### Example 1: Preparation of the compounds

### Compounds 1-6 were prepared as described in scheme 1:

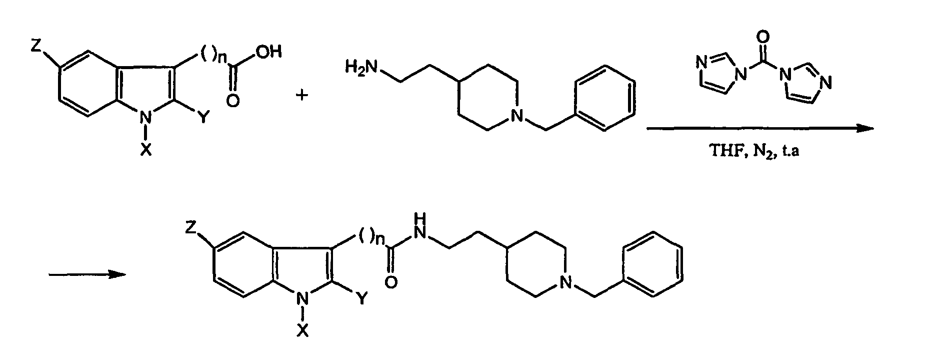

To a solution of indole-carboxilic acid derivates in THF, 1,1' carbonyldiimidazol was added under N₂, and the mixture was stirred for 4 hours at room temperature, after this time the amine was added and the resulting solution was stirred for 20 hours, after this time the solvent was evaporated under reduced pressure and the residue purified by silica gel column chromatography using as eluent mixtures of solvents in the proportions indicated for each particular case.

### (1) N-[2-(1-Benzyl-piperidin-4-yl)-ethyl]-2-(1H-indol-3-yl)-acetamide

Reagents: Indole-3-acetic acid ( 152.4mg, 0.87mmol), THF 3ml, 1,1'carbonyldiimidazol (149.2 mg, 0.92 mmol), and 2-(1-Benzyl-piperidin-4-yl)-ethylamine ( 200mg, 0.92mmol).
Conditions: Room temperature, overnight. Purification: silica gel column chromatography using AcOEt/ MeOH ( 2:1). Yield: 110mg (35%).¹H-NMR (CDCl₃, 400MHz, δ ppm): (8.53, br, 1H) (7.50, d, 1H, *J*=7.6 Hz), (7.35, d, 1H, *J*=7.6), 7.26-7.17, m, 6H), (7.11-7.08, m, 2H), ( 5.65, m, 1H, NHCO), ( 3.70, s, 2H), ( 3.43, s, 2H), (3.16, c, 2H, *J*=6.8), (2.77-2.74, m, 2H), (1.81-1.75, m, 2H ), (1.48-1.45, m, 2H), (1.25-1.20, m, 2H), (1.31-1.06, m, 3H).
¹³C-NMR (CDCl₃): 171.4, 138.0, 136.4, 129.3, 128.1, 127.0, 126.39, 123.7, 122.6, 120.0, 118.7, 111.4, 109.0, 63.3, 53.5, 37.2, 36.0, 33.4, 33.2, 32.0.
ESI-MS[M+H⁺]⁺375.23

### (2) N-[2-(1-Benzyl-piperidin-4-yl)-ethyl]-2-(1-methyl-1H-indol-3-yl)-acetamide

Reagents: 1-methylindole-3-carboxilic acid (165mg, 0.87mmol), THF 3ml, 1,1'carbonyldiimidazol ( 149.2 mg, 0.92 mmol), and 2-(1-Benzyl-piperidin-4-yl)-ethylamine ( 200mg, 0.92mmol).
Conditions: Room temperature, overnight. Purification: silica gel column chromatography using AcOEt/ MeOH ( 3:1). Yield: 40mg (12%).¹H-NMR (CDCl₃, 400MHz, δ ppm): (7.42, d, 1H, *J*=7.6 Hz), (7.25-7.13, m, 7H), (7.11-7.08, m, 1H), (6.70, s, 1H), ( 5.48, m, 1H, NHCO), ( 3.70, s, 3H), ( 3.60, s, 2H), (3.37, s, 2H), (3.16, m, 2H), (2.75-2.68, m, 2H), (1.70-1.80, m, 2H ), (1.40-1.48, m, 2H), ( 1.43-1.10, m, 2H), (1.10-1.01, m, 3H).
¹³C-NMR (CDCl₃): 171.4, 137.0, 129.3, 129.2, 128.3, 128.1, 127.35, 126.9, 122.1, 119.5, 118.8, 109.4, 107.4, 63.3, 53.5, 37.1, 36.0, 33.2, 32.3, 32.0.
ESI-MS[M+H⁺]⁺389.25

### (3) N-[2-(1-Benzyl-piperidin-4-yl)-ethyl]-2-(2-methyl-1H-indol-3-yl)-acetamide

Reagents: 2-methylindole-3-carboxilic acid (165mg, 0.87mmol), THF 3ml, 1,1'carbonyldiimidazol ( 149.2 mg, 0.92 mmol), and 2-(1-Benzyl-piperidin-4-yl)-ethylamine ( 200mg, 0.92mmol).
Conditions: Room temperature, overnight. Purification: silica gel column chromatography using AcOEt/ MeOH ( 5:.02). Yield: 153mg (46%).¹H-NMR (CDCl₃, 400MHz, δ ppm): (8.53, br, 1H), (7.42, d, 1H, *J*=7.6 Hz), (7.25-7.20, m, 5H), (7.18-7.01, m, 3H), ( 5.62, m, 1H, NHCO), ( 3.62, s, 2H), ( 3.42, s, 2H), (3.20-3.10, m, 2H), (2.80-2.72, m, 2H), (2.32, s, 3H), (1.82-1.75, m, 2H ), (1.5-1.44, m, 2H), (1.15-1.10, m, 2H) ( 1.20-1.05, m, 3H).
¹³C-NMR (CDCl₃): 171.5, 137.2, 129.2, 128.0, 126.9, 122.4, 119.7, 117.6, 110.5, 104.3, 63.3, 53.5, 37.0, 35.8, 33.2, 32.1, 31.9, 11.4.
ESI-MS[M+H⁺]⁺389.25

### (4) N-[2-(1-Benzyl-piperidin-4-yl)-ethyl]-2-(5-bromo-1H-indol-3-yl)-acetamide

Reagents: 5-bromoindole-3-acetic acid (221mg, 0.87mmol), THF 3ml, 1,1'carbonyldiimidazol ( 149.2 mg, 0.92 mmol), and 2-(1-Benzyl-piperidin-4-yl)-ethylamine ( 200mg, 0.92mmol).
Conditions: Room temperature, overnight. Purification: silica gel column chromatography using AcOEt/ MeOH ( 5:0.1). Yield: 60mg (15%).¹H-NMR (CDCl₃, 400MHz, δ ppm): (9.3, bs, 1H), (7.65, s, 1H), (7.29-7.20, m, 7H), (7.10, s, 1H), (5.71, m, 1H, NHCO), (3.65, s, 2H), (3.46, s, 2H), (3.25-3.17, m, 2H), (2.85-2.76, m, 2H), (1.88-1.80, m, 2H), (1.56-1.48, m, 2H ), (1.32-1.48, m, 2H), (1.10-1.01, m, 3H).
¹³C-NMR (CDCl₃): 170.6, 137.8, 134.7, 129.0, 128.4, 127.8, 126.6, 125.1, 124.6, 121.0, 112.9, 112.6, 108.3, 63.1, 53.2, 37.0, 35.6, 33.0, 32.9, 32.6.
ESI-MS[M+H⁺]⁺453.14.

### (5) N-[2-(1-Benzyl-piperidin-4-yl)-ethyl]-3-(1H-indol-3-yl)-propionamide

Reagents: 3-Indolepropionic acid (156mg, 0.82mmol), THF 3ml, 1,1'carbonyldiimidazol ( 141mg, 0.87mmol), and 2-(1-Benzyl-piperidin-4-yl)-ethylamine ( 188mg, 0.87mmol).
Conditions: Room temperature, overnight. Purification: silica gel column chromatography using AcOEt/ MeOH ( 1:0.1). Yield: 134.4mg (42%).¹H-NMR (CDCl₃, 400MHz, δ ppm): (8.85, s, 1H), (7.52, d, 1H, *J*=8 Hz), (7.25-7.20, m, 6H), (7.13-7.10, m, 1H), (7.06-7.02, s, 1H), (6.84, s, 1H), ( 5.67, m, 1H, NHCO), ( 3.45, s, 2H), ( 3.14-3.04, m, 4H), (2.81-2.78, m, 2H), (2.51-2.47, m, 2H), (1.87-1.81, m, 2H), (1.50-1.47, m, 2H ), (1.25-1.09, m, 5H).
¹³C-NMR (CDCl₃): 172.3, 137.9, 136.4, 129.4, 128.2, 127.0, 121.9, 121.8, 119.0, 118.6, 114.5, 111.4, 63.4, 53.6, 37.5, 37.3, 36.2, 33.4, 32.0, 21.7.
ESI-MS[M+H⁺]⁺389.25

### (6) N-[2-(1-Benzyl-piperidin-4-yl)-ethyl]-4-(1H-indol-3-yl)-butyramide

Reagents: 3-Indolebutyric acid (175.1mg, 0.86 mmol), THF 3ml, 1,1'carbonyldiimidazol ( 146.0 mg, 0.90 mmol), and 2-(1-Benzyl-piperidin-4-yl)-ethylamine ( 200mg, 0.92mmol).
Conditions: Room temperature, overnight. Purification: silica gel column chromatography using AcOEt/ MeOH ( 1:1). Yield: 95mg (30%).¹H-NMR (CDCl₃, 400MHz, δ ppm): (8.65, s, 1H), (7.53, d, 1H, *J*=7.6 Hz), (7.28-7.21, m, 6H), (7.14-7.10, m, 1H), (7.06-7.10, m, 1H), (6.70, m, 1H), ( 5.58, m, 1H, NHCO), ( 3.44, s, 3H), ( 3.18-3.13, m, 2H), (2.84-2.81, s, 2H), (2.76-2.72, m, 2H), (2.16-2.13, m, 2H), (2.01-1.97, m, 2H),(1.90-1.85, m, 2H ), (1.59-1.56, m, 2H), ( 1.34-1.18, m, 4H). ¹³C-NMR (CDCl₃): 173.1, 137.8, 136.3, 129.2, 129.1, 128.0, 127.33, 126.9, 121.6, 118.8, 118.6, 115.1, 111.2, 63.2, 53.5, 37.1, 36.1, 33.2, 32.0, 26.0, 24.4.
ESI-MS[M+H⁺]⁺403.26

### Compound (7) is prepared as described in scheme 2:

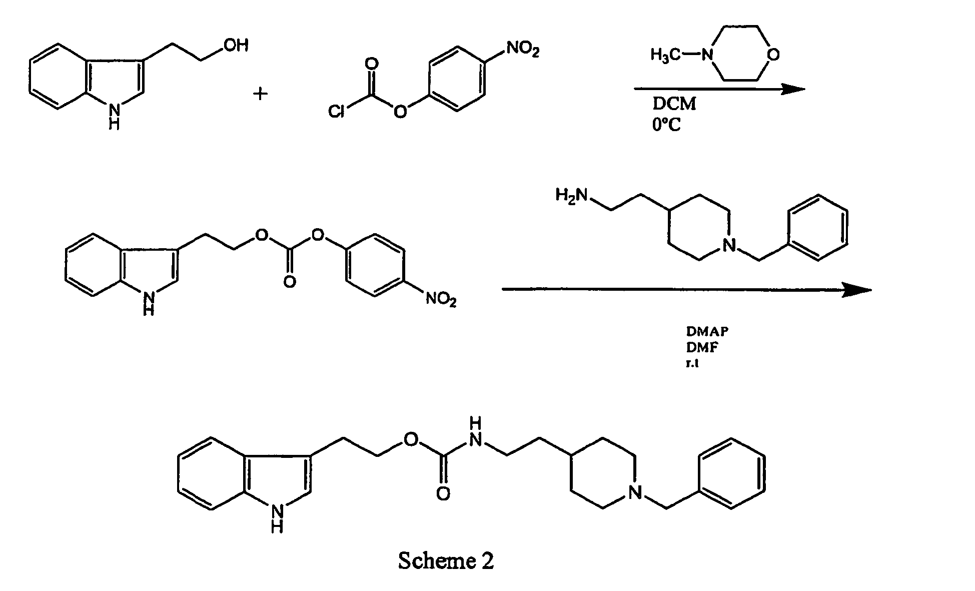

### (7) [2-(1-Benzyl-piperidin-4-yl)-ethyl]-carbamic acid 2-(1H-indol-3-yl)-ethyl ester

To a solution of 2-(1H-Indol-3-yl)-ethanol (500mg, 3.10mmol), in N-Methyl morpholine (627mg, 62mmol), was added *p*-nitrophenyl chloroformate (1250nmg, 6.2mmol), and the mixture was stirred for 24 hours at room temperature, after this time the residue was washed with water and extracted with dichloromethane after the residue was purified by silica gel column chromatography using as eluent mixture of DCM/Hx (3:1) 2-(1-Benzyl-piperidin-4-yl)-ethylamine (700mg, 3.21mmol), was dissolved with DMF and then coupled with activated carbonate (522mg, 1.60mmol) in presence of DMAP (40mg, 3.21mmol), the mixture was stirred for 24 hours at room temperature and then the solvent was evaporated under reduced pressure. After this time the residue was washed with water and extracted with dichloromethane after the residue was purified by silica gel column chromatography using as eluent mixture of DCM/MeOH (4:0.5) to obtain compound 7. Yield: 286mg (44%).¹H-NMR (CDCl₃, 400MHz, δ ppm): (8.26, bs, 1H), (7.59, m, 1H), (7.31-7.21, m, 5H), (7.18-7.10, m, 1H), (7.09-7.02, m, 1H), (6.80, m, 1H), ( 4.46, m, 1H, NHCO), ( 3.48, s, 2H), ( 3.12-3.10, m, 2H), (3.08-3.0, m, 2H), (2.90-2.84, m, 4H), (1.99-1.80, m, 2H), (1.52-1.5, m, 2H), (1.49-1.20, m, 5H ). ¹³C-NMR (CDCl₃): 157.0, 136.5, 129.6, 128.4, 127.2, 122.3, 122.2, 119.5, 119.0, 112.4, 111.4, 65.1, 63.5, 53.8, 38.9, 36.8, 33.4, 32.2, 25.5.
ESI-MS[M+H⁺]⁺405.24

### Example 2: Biological assays

### Butyrylcholinesterase (BuChE) inhibition (from human serum)

BuChE inhibitory activity was evaluated at 30°C by the colorimetric method reported by Ellman [Ellman, G.L.; Courtney, K.D.; Andres, B.; Featherstone, R.M. Biochem. Pharmacol. 1961, 7, 88-95]. The assay solution consisted of 0.1 unit/ml BuChE from human serum, 0.1 M sodium phosphate buffer pH 8, 0.3 mM 5,5'-dithiobis (2-nitrobenzoic acid) (DTNB, Ellman's reagent), and 0.5 mM butyrylthiocholine iodide as the substrate of the enzymatic reaction. Enzyme activity was determined by measuring the absorbance at 405 nm during 5 minutes with a microplate reader Digiscan 340T. The tested compounds were preincubated with the enzyme for 10 minutes at 30°C. The reaction rate was calculated with, at least, triplicate measurements. The IC₅₀ is defined as the concentration of each compound that reduces a 50% the enzymatic activity with respect to that without inhibitors. The results are shown in table 1.

### Acetylcholinesterase (AChE) Inhibition (from Bovine Erythrocytes)

AChE inhibitory activity was evaluated at 30°C by the colorimetric method reported by Ellman [Ellman, G.L.; Courtney, K.D.; Andres, B.; Featherstone, R.M. Biochem. Pharmacol. 1961, 7, 88-95]. The assay solution consisted of 0.1 M phosphate buffer pH 8, 0.3 mM 5,5'-dithiobis (2-nitrobenzoic acid) (DTNB, Ellman's reagent), 0.2 unit/ml AChE (Sigma Chemical Co. from bovine erythrocytes), and 0.5 mM acetylthiocholine iodide as the substrate of the enzymatic reaction. The compounds tested were added to the assay solution and pre incubated with the enzyme for 5 min at 30°C. After that period, the substrate was added. The absorbance changes at 405 nm were recorded for 5 min with a microplate reader Digiscan 340T, the reaction rates were compared, and the percent inhibition due to the presence of test compounds was calculated. The reaction rate was calculated with, at least, triplicate measurements, and the percent inhibition due to the presence of test compound was calculated relative to the compound-free control. The compound concentration producing 50% of AChE inhibition (IC₅₀) was determined. The results are shown in table 1.

### Toxicity measurement

The cytotoxicity effect of the molecules was tested in the human neuroblastoma cell line SH-SY5Y. These cells were cultured in 96-well plates in a 1:1 mixture of Ham's F12 nutrients and essential medium (MEM), supplemented with 10% fetal bovine serum and 1% penicillin/streptomycin, and grown in a 5% CO₂ humidified incubator at 37°C.
The cells were plated at 10⁴ cells for each well, at least, 48 hours before the toxicity measure. Cells were exposed for 24 hours to the compounds at different concentrations (from 10⁻⁵ to 10⁻⁹), quantitative assessment of cell death was made by measurement of the intracellular enzyme lactate dehydrogenase (LDH) (citotoxicity detection kit, Roche). The quantity of LDH was measured and evaluated in a microplate reader Anthos 2010, at 492 and 620 nm. Controls were taken as 100% viability. The results are shown in table 1.

### Propidium competition

Propidium exhibits an increase in fluorescence on binding to AChE peripheral site, making it a useful probe for competitive ligand binding to the enzyme.
Fluorescence was measured in a Fluostar optima plate reader (BMG). Measurements were carried out in 100 µl solution volume, in 96-well plates. The buffer used was 1 mM Tris/HCl, pH 8.0. 5 µM AchE was incubated, at least 6 hours, with the molecules at different concentrations. 20 µM propidium iodide was added 10 min before fluorescence measurement. The excitation wavelength was 485 nm, and that of emission, 620 nm. The results are shown in table 1.

**Table 1**

| **compound** | **structure** | **IC₅₀ BuChE (nM)** | **IC₅₀ AChE (nM)** | **Toxicity (µM)** | **Propidium competition (**µ**M)** |
|---|---|---|---|---|---|
| **1** | 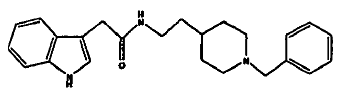 | 0.8 | 200 | >100 | 1 |
| **2** | 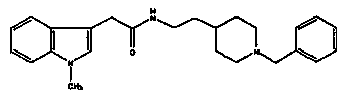 | 1 | 400 | >100 | 100 |
| **3** | 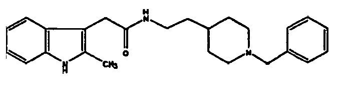 | 2 | 200 | >100 | 100 |
| **4** | 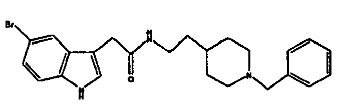 | 0.25 | 250 | >100 | 1 |
| **5** | 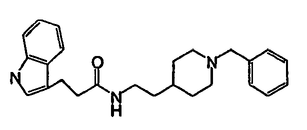 | 0.05 | 2000 | >100 | 100 |
| **6** | 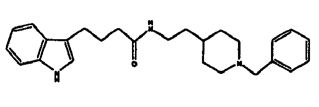 | 5 | 300 | >100 | 100 |
| **7** | 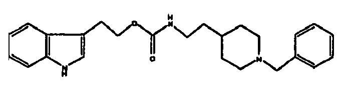 | 30 | 1000 | >100 | 10 |

It can be appreciated from the results that very high BuChE inhibition activities have been achieved. The selectivity with respect to AChE is at least of two orders of magnitude, in the case of compound 5 it is of 5.

## Claims

1. A compound of formula I: 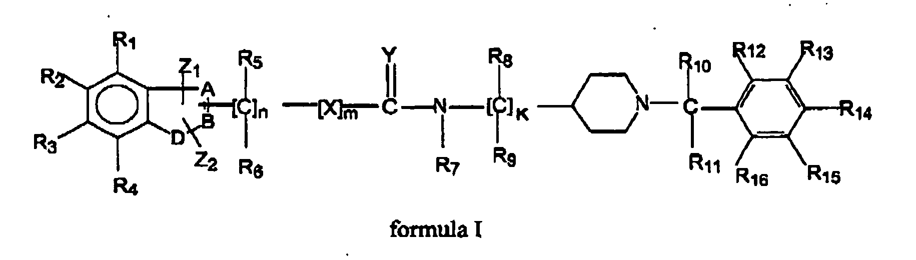 wherein
A,B are independently selected from C or N;
D is selected fom C, O, S, N;
With the proviso that at least one of A, B and D is an heteroatom;
X is selected from -CRₐR_{b}-, -O-, -S-, -NRₐ-;
Y is selected from O, S, NRₐ;
Z₁ and Z₂ are independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, -COR₃, -C(O)ORₐ,-C(O)NRₐR_{b}, -C=NRₐ, -CN, -ORₐ, -OC(O)Rₐ, -NRₐR_{b}, -NRₐC(O)R_{b}, -NO₂, -N=CRₐR_{b}, or halogen,
wherein Z₁ and Z₂ together with A and B or B and D can form a fused ring system, or together with R₁ or R₄ they can form a fused ring system;
R₁ to R₁₆ are independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, -CORₐ, -C(O)ORₐ,-C(O)NRₐR_{b}, -C=NRₐ, -CN, -ORₐ, -OC(O)Rₐ, -S(O)ₜ-Rₐ, -NRₐR_{b}, -NRₐC(O)R_{b}, -NO₂,-N=CRₐR_{b} or halogen;
wherein Rₐ and R_{b} are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy or halogen; n is 1 to 6;
m is 0 or 1;
k is 1-8;
or a pharmaceutically acceptable salt or solvate thereof;
with the proviso that the compound is not (αR) 1H-Indole-3-propanamide, N-[[1-[(4-chlorophenyl)methyl]-4piperidinyl]methyl]-α-[(3-ethoxybenzoyl)amino].

2. A compound according to claim 1 which has the following formula II: 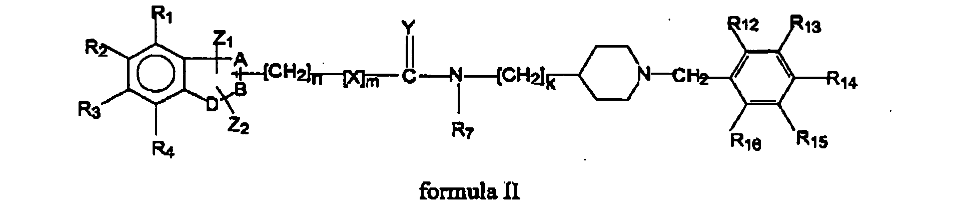 wherein A,B,D, Z₁, Z₂, X, Y, n, m, k, and R₁ to R₁₆ are as defined in claim 1, or a pharmaceutically acceptable salt or solvate thereof.

3. A compound according to claim 1 which has the following formula III: 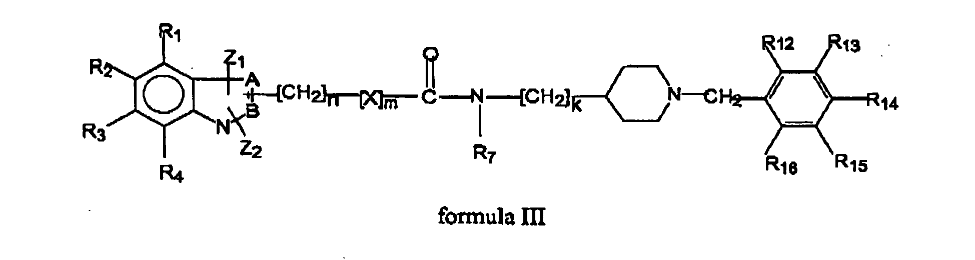 wherein A, B, Z₁, Z₂, X, n, m, k, and R₁ to R₁₆ are as defined in claim 1, or a pharmaceutically acceptable salt or solvate thereof .

4. A compound according to claim 1 which has the following formula IV: 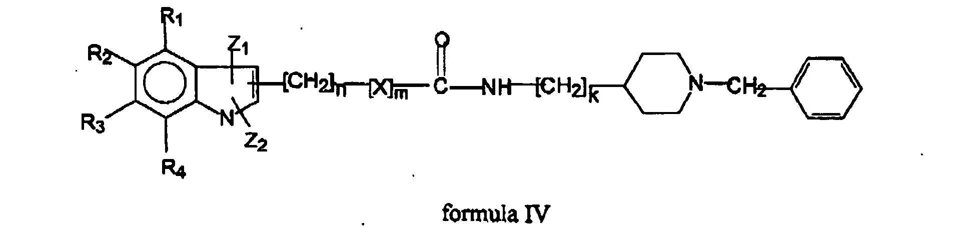 wherein Z₁, Z₂, X, n, m, k, and R₁ to R₄ are as defined in claim 1, or a pharmaceutically acceptable salt or solvate thereof.

5. A compound according to any of claims 1-4 wherein m is 1 and X is -CH₂- or -0-, or a pharmaceutically acceptable salt or solvate thereof.

6. A compound according to any of claims 1-5 wherein k is 2, or a pharmaceutically acceptable salt or solvate thereof.

7. A compound according to any of claims 1-6, wherein m is 0 and n is 2, or a pharmaceutically acceptable salt or solvate thereof.

8. A pharmaceutical composition which comprises a compound according to formula (I) or a pharmaceutically acceptable salt or solvate thereof, as claimed in any of claims 1-7 and a pharmaceutically acceptable carrier, adjuvant or vehicle.

9. A pharmaceutical composition according to claim 8 for oral administration.

10. Use of a compound as defined in any of claims 1-9 in the manufacture of a medicament.

11. Use according to claim 10 wherein the medicament is for the treatment of cognitive disorders as senile dementia, cerebrovascular dementia, mild recognition impairment, attention deficit disorder, and /or neurodegenerative dementing disease with aberrant protein aggregations as specially Alzheimers's disease or condition, or prion disease as Creutzfeld-Jacob disease or Gerstmann-Straussler-Scheinher disease.

12. Use according to claim 10 wherein the medicament is for the treatment of Alzheimers's disease or condition.

13. Use of the compounds of as defined in any of claims 1-7 as reactives for biological assays.

14. A process for the preparation of a compound as defined in any of claims 1-7 which comprises the reaction of the two compounds: 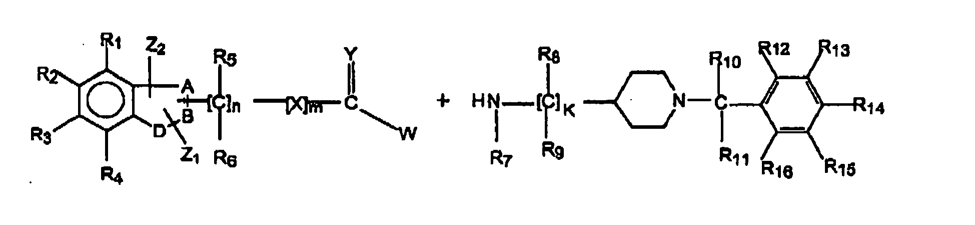 wherein A,B,D, Z₁, Z₂, X, Y, n, m, k, and R₁ to R₁₆ are as defined in claim 1 and W is a leaving group.

## Patentansprüche

1. Verbindung der Formel I: 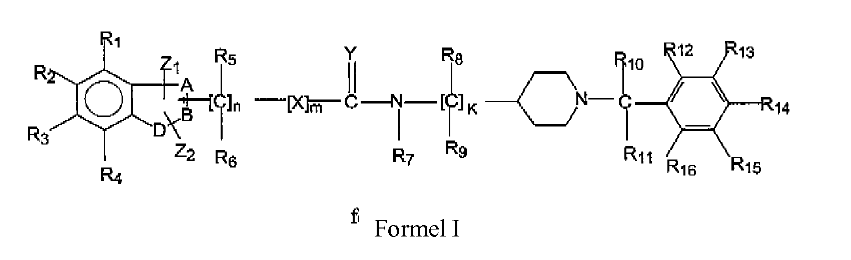 wobei
A, B unabhängig ausgewählt sind aus C oder N;
D ausgewählt ist aus C, O, S, N;
mit der Maßgabe, dass mindestens eines aus A, B und D ein Heteroatom ist;
X ausgewählt ist aus -CRₐ-R_{b}-, -O-, -S-, -NRₐ-;
Y ausgewählt ist aus O, S, NRₐ;
Z₁ und Z₂ unabhängig ausgewählt sind aus Wasserstoff, substituiertem oder unsubstituiertem Alkyl, substituiertem oder unsubstituiertem Cycloalkyl, substituiertem oder unsubstituiertem Alkenyl, substituiertem oder unsubstituiertem Aryl, substituiertem oder unsubstituiertem Heterocyclyl, -CORₐ, -C(O)ORₐ,-C(O)NRₐR_{b}, -C=NRₐ, -CN, -ORₐ, -OC(O)Rₐ, -NRₐR_{b}, -NRₐC(O)R_{b}, -NO₂, -N=CRₐR_{b} oder Halogen,
wobei Z₁ und Z₂ zusammen mit A und B oder B und D ein kondensiertes Ringsystem bilden können oder zusammen mit R₁ oder R₄ ein kondensiertes Ringsystem bilden können;
R₁ bis R₁₆ unabhängig ausgewählt sind aus Wasserstoff, substituiertem oder unsubstituiertem Alkyl, substituiertem oder unsubstituiertem Cycloalkyl, substituiertem oder unsubstituiertem Alkenyl, substituiertem oder unsubstituiertem Aryl, substituiertem oder unsubstituiertem Heterocyclyl, -CORₐ, -C(O)ORₐ,-C(O)NRₐR_{b}, -C=NRₐ, -CN, -ORₐ, -OC(O)Rₐ, -S(O)ₜ-Rₐ, -NRₐR_{b}, -NRₐC(O)R_{b}, -NO₂, -N=CRₐR_{b} oder Halogen;
wobei Rₐ und R_{b} jeweils unabhängig ausgewählt sind aus Wasserstoff, substituiertem oder unsubstituiertem Alkyl, substituiertem oder unsubstituiertem Cycloalkyl, substituiertem oder unsubstituiertem Alkenyl, substituiertem oder unsubstituiertem Aryl, substituiertem oder unsubstituiertem Heterocyclyl, substituiertem oder unsubstituiertem Alkoxy, substituiertem oder unsubstituiertem Aryloxy oder Halogen;
n 1 bis 6 ist;
m 0 oder 1 ist;
k 1 bis 8 ist;
oder ein pharmazeutisch verträgliches Salz oder Solvat davon;
mit der Maßgabe, dass die Verbindung nicht (αR)-1H-Indol-3-propanamid, N-[[1-[(4-chlorphenyl)methyl]-4-piperidinyl]methyl]-α-[(3-ethoxybenzoyl)amino] ist.

2. Verbindung gemäß Anspruch 1, welche die folgende Formel II aufweist: 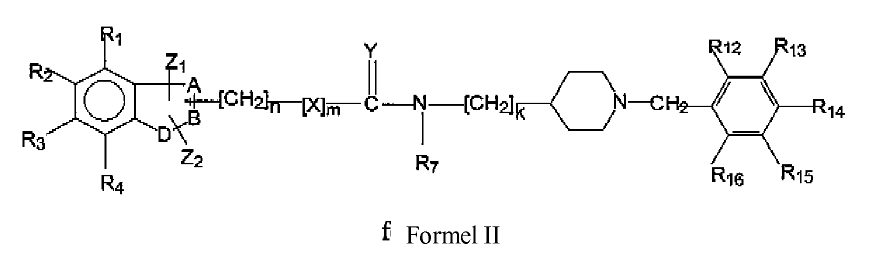 wobei A, B, D, Z₁, Z₂, X, Y, n, m, k und R₁ bis R₁₆ wie in Anspruch 1 definiert sind, oder ein pharmazeutisch verträgliches Salz oder Solvat davon.

3. Verbindung gemäß Anspruch 1, welche die folgende Formel III aufweist: 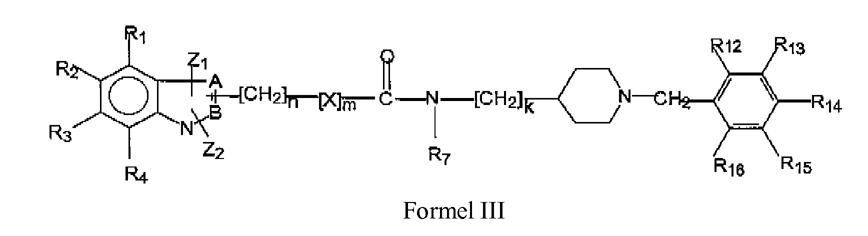 wobei A, B, Z₁, Z₂, X, n, m, k und R₁ bis R₁₆ wie in Anspruch 1 definiert sind, oder ein pharmazeutisch verträgliches Salz oder Solvat davon.

4. Verbindung gemäß Anspruch 1, welche die folgende Formel IV aufweist: 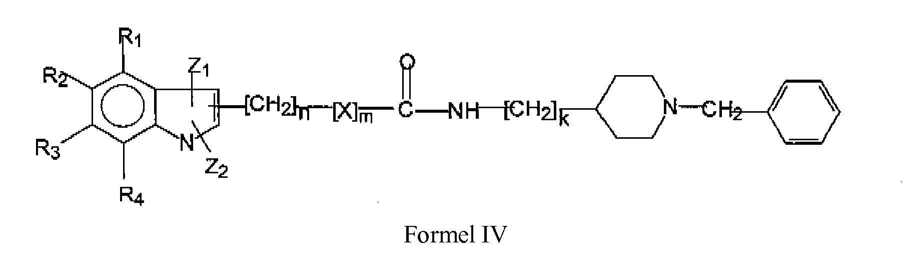 wobei Z₁, Z₂, X, n, m, k und R₁ bis R₄ wie in Anspruch 1 definiert sind, oder ein pharmazeutisch verträgliches Salz oder Solvat davon.

5. Verbindung gemäß einem der Ansprüche 1 bis 4, wobei m 1 ist und X -CH₂- oder -O- ist, oder ein pharmazeutisch verträgliches Salz oder Solvat davon.

6. Verbindung gemäß einem der Ansprüche 1 bis 5, wobei k 2 ist, oder ein pharmazeutisch verträgliches Salz oder Solvat davon.

7. Verbindung gemäß einem der Ansprüche 1 bis 6, wobei m 0 ist und n 2 ist, oder ein pharmazeutisch verträgliches Salz oder Solvat davon.

8. Pharmazentische Zusammensetzung, welches eine Verbindung gemäß Formel (I) oder ein pharmazeutisch verträgliches Salz oder Solvat davon gemäß einem der Ansprüche 1 bis 7 und ein(en) pharmazeutisch verträgliches(n) Träger, Hilfsstoff oder Vehikel umfasst.

9. Pharmazentische Zusammensetzung gemäß Anspruch 8 zur oralen Verabreichung.

10. Verwendung einer Verbindung wie in einem der Ansprüche 1 bis 9 definiert zur Herstellung eines Medikaments.

11. Verwendung gemäß Anspruch 10, wobei das Medikament für die Behandlung von kognitiven Störungen wie seniler Demenz, cerebrovaskulärer Demenz, schwacher Erkennungsbeeinträchtigung, Aufmerksamkeitsdefizit-Syndrom und/oder neurodegenerativer Demenzerkrankung mit anormalen Proteinaggregationen, wie insbesondere die Alzheimer-Krankheit oder der Alzheimer-Zustand, oder Prionenerkrankungen, wie die Creutzfeldt-Jacob-Krankheit oder das Gerstmann-Sträussler-Scheinher-Syndrom, bestimmt ist.

12. Verwendung gemäß Anspruch 10, wobei das Medikament für die Behandlung der Alzheimer-Krankheit oder des Alzheimer-Zustands bestimmt ist.

13. Verwendung der Verbindungen wie in einem der Ansprüche 1 bis 7 definiert als Reaktanten für biologische Tests.

14. Verfahren zur Herstellung einer Verbindung wie in einem der Ansprüche 1 bis 7 definiert, welches die Umsetzung der zwei Verbindungen umfasst: 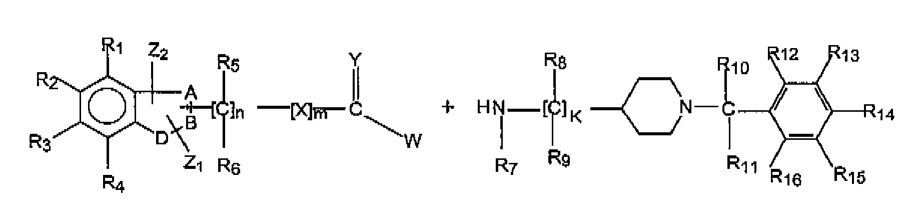 wobei A, B, D, Z₁, Z₂, X, Y, n, m, k und R₁ bis R₁₆ wie in Anspruch 1 definiert sind und W eine Abgangsgruppe ist.

## Revendications

1. Composé de formule I : 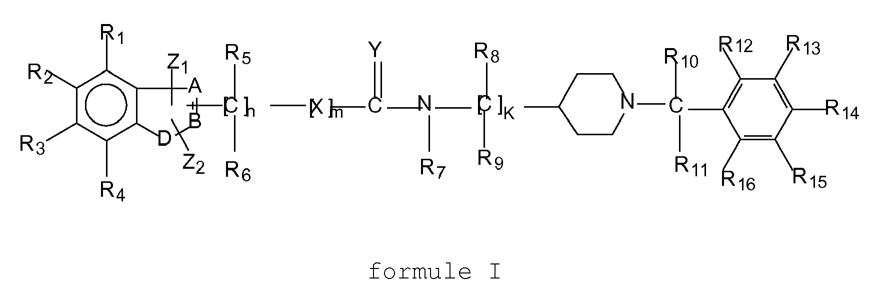 dans lequel
A, B sont choisis indépendamment parmi C et N,
D est choisi parmi C, O, S, N,
avec la condition que parmi A, B et D, au moins un est un hétéroatome,
X est choisi parmi -CRₐR_{b}-, -O-, -S-, -NRₐ-,
Y est choisi parmi O, S, NRₐ,
Z₁ et Z₂ sont choisis indépendamment parmi l'hydrogène, un alkyle substitué ou non, un cycloalkyle substitué ou non, un alkényle substitué ou non, un aryle substitué ou non, un hétérocyclyle substitué ou non, -CORₐ, -C(O)ORₐ,-C(O)NRₐR_{b}, -C=NRₐ, -CN, -ORₐ, -OC(O)Rₐ, -NRₐR_{b}, -NRₐC(O)R_{b},-NO₂, -N=CRₐR_{b}, ou un halogène,
dans lequel Z₁ et Z₂ avec A et B ou B et D peuvent former un système d'anneau fusionné, ou avec R₁ ou R₄ ils peuvent former un système d'anneau fusionné,
R₁ à R₆ sont choisis indépendamment parmi l'hydrogène, un alkyle substitué ou non, un cycloalkyle substitué ou non, un alkényle substitué ou non, un aryle substitué ou non, un hétérocyclyle substitué ou non, -CORₐ, -C(O)ORₐ,-C(O)NRₐR_{b}, -C=NRₐ, -CN, -ORₐ, -OC(O)Rₐ, -S(O)ₜ-Rₐ, -NRₐR_{b},-NRₐC(O)R_{b}, -NO₂, -N=CRₐR_{b}, ou un halogène,
dans lequel Rₐ et R_{b} sont chacun choisis indépendamment parmi l'hydrogène, un alkyle substitué ou non, un cycloalkyle substitué ou non, un alkényle substitué ou non, un aryle substitué ou non, un hétérocyclyle substitué ou non, un alcoxyle substitué ou non, un aryloxyle substitué ou non ou un halogène,
n est compris entre 1 et 6,
m est égal à 0 ou 1,
k est compris entre 1 et 8,
ou un sel ou solvate pharmaceutiquement acceptable de celui-ci,
avec la condition que le composé n'est pas (αR) 1H-Indole-3-propanamide, N-[[1-[(4-chlorophényle)méthyle]-4pipéridinyle]méthyle]-α-[(3-éthoxybenzoyl)amino].

2. Composé selon la revendication 1 qui a la formule II suivante : 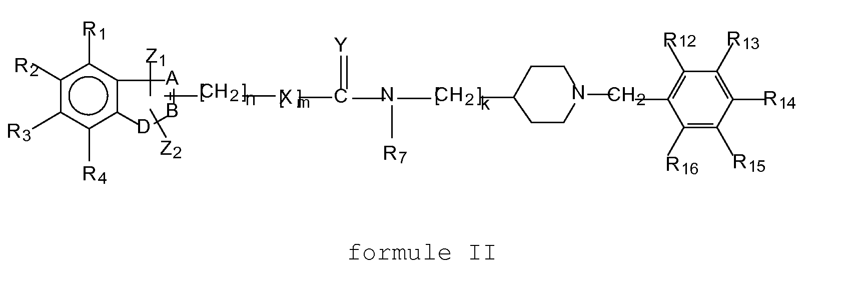 **caractérisé en ce que** A, B, D, Z₁, Z₂, X, Y, n, m, k et R₁ à R₁₆ sont tels que définis dans la revendication 1, ou un sel ou solvate pharmaceutiquement acceptable de celui-ci.

3. Composé selon la revendication 1 qui a la formule III suivante : 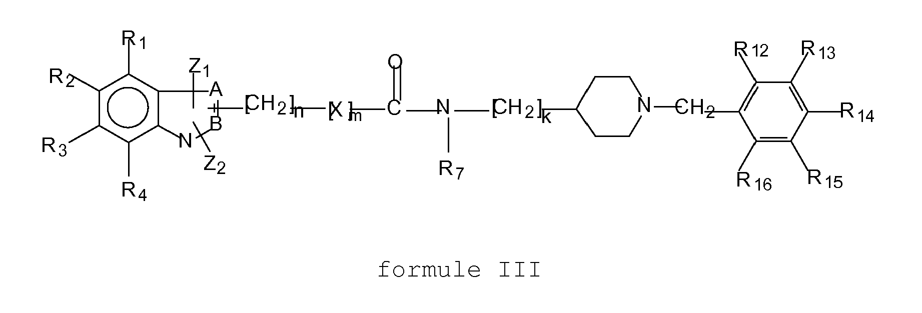 **caractérisé en ce que** A, B, Z₁, Z₂, X, n, m, k et R₁ à R₁₆ sont tels que définis dans la revendication 1, ou un sel ou solvate pharmaceutiquement acceptable de celui-ci.

4. Composé selon la revendication 1 qui a la formule IV suivante : 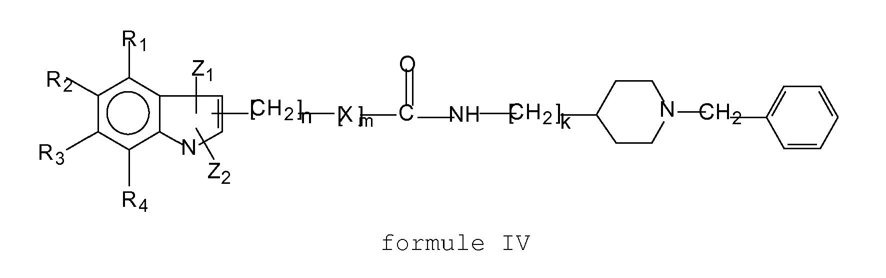 **caractérisé en ce que** Z₁, Z₂, X, n, m, k et R₁ à R₄ sont tels que définis dans la revendication 1, ou un sel ou solvate pharmaceutiquement acceptable de celui-ci.

5. Composé selon l'une quelconque des revendications 1 à 4, **caractérisé en ce que** m est égal à 1 et X est -CH₂ ou -O- ou un sel ou solvate pharmaceutiquement acceptable de celui-ci.

6. Composé selon l'une quelconque des revendications 1 à 5, **caractérisé en ce que** k est égal à 2 ou un sel ou solvate pharmaceutiquement acceptable de celui-ci.

7. Composé selon l'une quelconque des revendications 1 à 6, **caractérisé en ce que** m est égal à 0 et n est égal à 2 ou un sel ou solvate pharmaceutiquement acceptable de celui-ci.

8. Composition pharmaceutique comprenant un composé selon la formule (I) ou un sel ou solvate pharmaceutiquement acceptable de celui-ci, comme revendiqué dans l'une quelconque des revendications 1 à 7 et un porteur, adjuvant ou véhicule pharmaceutiquement acceptables.

9. Composition pharmaceutique selon la revendication 8 destinée à l'administration orale.

10. Utilisation d'un composé tel que défini dans l'une quelconque des revendications 1 à 9 dans la fabrication d'un médicament.

11. Utilisation selon la revendication 10, **caractérisée en ce que** le médicament est destiné au traitement des désordres cognitifs comme la démence sénile, la démence cérébrovasculaire, la détérioration légère de la récognition, le trouble déficitaire de l'attention, et/ou la maladie neurodégénérative de démence avec des agrégations de protéines aberrantes comme en particulier la condition au maladie d'Alzheimer ou la maladie du prion comme la maladie de Creutzfeld-Jacob ou la maladie de Gerstmann-Straussler-Scheinher.

12. Utilisation selon la revendication 10, **caractérisée en ce que** le médicament est destiné au traitement de la condition au maladie d'Alzheimer.

13. Utilisation des composés tels que définis dans l'une quelconque des revendications 1 à 7 comme réactifs pour des essais biologiques.

14. Procédé pour la préparation d'un composé tel que défini dans l'une quelconque des revendications 1 à 7 qui comprend la réaction des deux composés : 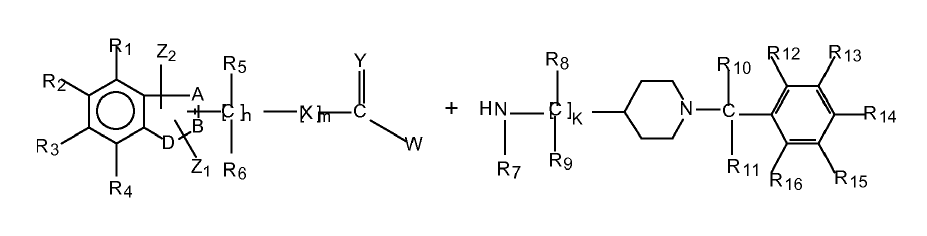 **caractérisé en ce que** A, B, D, Z₁, Z₂, X, Y, n, m, k et R₁ à R₁₆ sont tels que définis dans la revendication 1 et W est un groupe détachable.
